# FASCICULE DE BREVET EUROPEEN

(11) **EP 1 190 257 B1**
(45) Date de publication et mention de la délivrance du brevet: **25.08.2004**
(21) Numéro de dépôt: 00951579.2
(22) Date de dépôt: 09.06.2000
(51) Int. Cl.: G01N 33/569, C07K 14/18, A61K 39/12, A61K 48/00, A61K 39/395

(54) **DETECTION PRECOCE DES FLAVIVIRUS EN UTILISANT LA GLYCOPROTEINE NS1**
FRÜHERKENNUNG DER FLAVIVIREN UNTER VERWENDUNG DES NS1-GLYCOPROTEINS
EARLY DETECTION OF FLAVIVIRUSES USING THE NS1 GLYCOPROTEIN

(30) Priorité: 09.06.1999 FR 9907290; 10.06.1999 FR 9907361
(43) Date de publication de la demande: 27.03.2002
(73) Titulaire: INSTITUT PASTEUR, 75724 Paris Cédex 15 (FR)
(72) Inventeur: FLAMAND, Marie, F-75013 Paris (FR); MEGRET, Françoise, F-75016 Paris (FR); ALCON, Sophie, F-93190 Livry-Gargan (FR); TALARMIN, Antoine, 75724 Paris Cedex 15 (FR); DESPRES, Philippe, F-92250 Garenne-Colombes (FR); DEUBEL, Vincent, F-92170 Vanves (FR)
(74) Mandataire: Goulard, Sophie
(86) Numéro de dépôt international: PCT/FR2000/001620
(87) Numéro de publication internationale: WO 2000/075665

(56) Documents cités:
- EP-A- 0 402 116
- WO-A-93/22440
- CROOKS AJ, LEE JM, EASTERBROOK LM, TIMOFEEV AV, STEPHENSON JR: "The NS1 protein of tick-borne encephalitis virus forms multimeric species upon secretion from the host cell" JOURNAL OF GENERAL VIROLOGY, vol. 75, no. 12, décembre 1994 (1994-12), pages 3453-3460, XP002132937 cité dans la demande
- A FALCONAR ET AL: "Precise location of sequential dengue virus subcomplex and complex B cell epitopes on the nonstructural-1 glycoprotein" ARCHIVES OF VIROLOGY,US,NEW YORK, NY, vol. 137, no. 3/04, 1994, pages 315-326-326, XP002101238 ISSN: 0304-8608 cité dans la demande
- FALCONAR AK, YOUNG PR: "Production of dimer-specific and dengue virus group cross-reactive mouse monoclonal antibodies to the dengue 2 virus non-structural glycoprotein NS1" JOURNAL OF GENERAL VIROLOGY, vol. 72, no. 4, avril 1991 (1991-04), pages 961-965, XP000890192
- FALCONAR AK, YOUNG PR: "Immunoaffinity purification of native dimer forms of the flavivirus non-structural glycoprotein, NS1" JOURNAL OF VIROLOGICAL METHODS, vol. 30, no. 3, décembre 1990 (1990-12), pages 323-332, XP000879370 cité dans la demande
- JACOBS SC, STEPHENSON JR, WILKINSON GW: "High-level expression of the tick-borne encephalitis virus NS1 protein by using an adenovirus-based vector: protection elicited in a murine model" JOURNAL OF VIROLOGY, vol. 66, no. 4, avril 1992 (1992-04), pages 2086-2095, XP000884360 cité dans la demande
- FLAMAND M, CHEVALIER M, HENCHAL E, GIRARD M, DEUBEL V: "Purification and renaturation of Japanese encephalitis virus nonstructural glycoprotein NS1 overproduced by insect cells" PROTEIN EXPRESSION AND PURIFICATION, vol. 6, no. 4, août 1995 (1995-08), pages 519-527, XP000879369
- HALL R A ET AL: "Immunoaffinity purification of the NS1 protein of Murray Valley encephalitis virus: selection of the appropriate ligand and optimal conditions for elution." JOURNAL OF VIROLOGICAL METHODS, (1991 APR) 32 (1) 11-20., XP000890099

### LISTE DE SEQUENCES

<110> INSTITUT PASTEUR
   FLAMAND Marie
   MEGRET Françoise
   ALCON Sophie
   TALARMIN Antoine
   DESPRES Philippe
   DEUBEL Vincent
<120> METHODE DE DETECTION PRECOCE DES FLAVIVIRUS ET SES APPLICATIONS
<130> S228FR78B
<140>
   <141>
<150> FR9907290
   <151> 1999-06-09
<160> 2
<170> PatentIn Ver. 2.1
<210> 1
   <211> 1137
   <212> ADN
   <213> Dengue virus type 1 NS1 protein
<400> 1
   atgaggagcg cgtcgctttc gatgacgtgc attgcagttg gcatggttac actgtaccta 60
   ggagtcatgg ttcaagcgga ctcgggatgt gtaatcaact ggaagggcag agaactcaaa 120
   tgtggaagtg gcatttttgt cactaatgaa gtccacactt ggacagagca atacaaattc 180
   caggctgact ccccaaaaag actgtcagca gccattggga aggcatggga ggagggcgtg 240
   tgtggaattc gatcagccac gcgtcttgag aacatcatgt ggaagcaaat atcaaatgaa 300
   ttgaaccaca ttctacttga aaatgacatg aaattcacag tggttgtagg agatgctaat 360
   ggaattttgg cccaggggaa aaaaatgatc aggccacaac ccatggaaca caaatactca 420
   tggaaaagct ggggaaaagc caagatcata ggagcagaca cacagaatac caccttcatc 480
   atcgacggcc cagacactcc agaatgcccc gatgaccaaa gagcgtggaa catttgggaa 540
   gttgaggact atgggtttgg aattttcacg acaaacatat ggctgaaatt gcgtgactcc 600
   tacacccaaa tgtgtgacca ccggctaatg tcagctgccg tcaaggacag caaggcagtc 660
   catgctgaca tggggtactg gatagaaagt gaaaagaacg agacctggaa gctagcgaga 720
   gcctccttca tagaagtcaa gacatgcatt tggccgaaat cccacactct atggagtaat 780
   ggagttttgg aaagtgaaat gataatccca aagatatatg gaggaccaat atctcagcac 840
   aattacagac cagggtattt cacacaaaca gcagggccat ggcacctagg taagttggaa 900
   ttggattttg acttgtgtga aggcaccaca gttgttgtgg atgaacattg tggaaatcga 960
   ggtccatctc tcagaactac aacagtcaca ggaaagataa tccatgaatg gtgttgcaga 1020
   tcctgcacgt tacccccctt acgcttcaga ggagaagacg gatgttggta tggcatggaa 1080
   atcagaccag ttaaggagaa ggaggagaac ctagttaggt caatggtctc tgcataa    1137
<210> 2
   <211> 378
   <212> PRT
   <213> Dengue virus type 1 NS1 protein
<400> 2
   Met Arg Ser Ala Ser Leu Ser Met Thr Cys Ile Ala Val Gly Met Val
    1               5                  10                  15
   Thr Leu Tyr Leu Gly Val Met Val Gln Ala Asp Ser Gly Cys Val Ile
   20 25 30
   Asn Trp Lys Gly Arg Glu Leu Lys Cys Gly Ser Gly Ile Phe Val Thr
   35 40 45
   Asn Glu Val His Thr Trp Thr Glu Gln Tyr Lys Phe Gln Ala Asp Ser
   50 55 60
   Pro Lys Arg Leu Ser Ala Ala Ile Gly Lys Ala Trp Glu Glu Gly Val
   65 70 75 80
   Cys Gly Ile Arg Ser Ala Thr Arg Leu Glu Asn Ile Met Trp Lys Gln
   85 90 95
   Ile Ser Asn Glu Leu Asn His Ile Leu Leu Glu Asn Asp Met Lys Phe
   100 105 110
   Thr Val Val Val Gly Asp Ala Asn Gly Ile Leu Ala Gln Gly Lys Lys
   115 120 125
   Met Ile Arg Pro Gln Pro Met Glu His Lys Tyr Ser Trp Lys Ser Trp
   130 135 140
   Gly Lys Ala Lys Ile Ile Gly Ala Asp Thr Gln Asn Thr Thr Phe Ile
   145 150 155 160
   Ile Asp Gly Pro Asp Thr Pro Glu Cys Pro Asp Asp Gln Arg Ala Trp
   165 170 175
   Asn Ile Trp Glu Val Glu Asp Tyr Gly Phe Gly Ile Phe Thr Thr Asn
   180 185 190
   Ile Trp Leu Lys Leu Arg Asp Ser Tyr Thr Gln Met Cys Asp His Arg
   195 200 205
   Leu Met Ser Ala Ala Val Lys Asp Ser Lys Ala Val His Ala Asp Met
   210 215 220
   Gly Tyr Trp Ile Glu Ser Glu Lys Asn Glu Thr Trp Lys Leu Ala Arg
   225 230 235 240
   Ala Ser Phe Ile Glu Val Lys Thr Cys Ile Trp Pro Lys Ser His Thr
   245 250 255
   Leu Trp Ser Asn Gly Val Leu Glu Ser Glu Met Ile Ile Pro Lys Ile
   260 265 270
   Tyr Gly Gly Pro Ile Ser Gln His Asn Tyr Arg Pro Gly Tyr Phe Thr
   275 280 285
   Gln Thr Ala Gly Pro Trp His Leu Gly Lys Leu Glu Leu Asp Phe Asp
   290 295 300
   Leu Cys Glu Gly Thr Thr Val Val Val Asp Glu His Cys Gly Asn Arg
   305 310 315 320
   Gly Pro Ser Leu Arg Thr Thr Thr Val Thr Gly Lys Ile Ile His Glu
   325 330 335
   Trp Cys Cys Arg Ser Cys Thr Leu Pro Pro Leu Arg Phe Arg Gly Glu
               340                 345                 350
   Asp Gly Cys Trp Tyr Gly Met Glu Ile Arg Pro Val Lys Glu Lys Glu
           355                 360                 365
   Glu Asn Leu Val Arg Ser Met Val Ser Ala
       370                 375

## Description

La présente invention est relative à une méthode de détection précoce des flavivirus, notamment du virus de la dengue et à ses applications.

La dengue est une maladie tropicale aiguë fébrile dont le virus causal est un arbovirus, transmis par des moustiques. Les vecteurs de la maladie sont des moustiques du genre *Aedes,* notamment *Aedes aegypti* dont les gîtes larvaires sont le plus souvent domestiques et péridomestiques. Le virus responsable, isolé en 1951, a été classé en quatre types antigéniques différents (DEN1, DEN2, DEN3 et DEN4). Il appartient à la famille des *Flaviviridae,* genre flavivirus.

Plus de deux milliards d'habitants vivent dans les zones endémiques et le nombre d'individus infectés par le virus s'élèverait à plus de 100 millions par an. La dengue est notamment responsable de 500 000 hospitalisations et de plusieurs dizaines de milliers de décès annuels, des enfants, pour la plupart.

Après une incubation de cinq à huit jours, le début des signes cliniques est généralement soudain et consiste en l'apparition de fièvre non différenciée (DF *dengue fever*) accompagnée de céphalées sévères, de lombalgies, de douleurs musculaires et articulaires ainsi que de frissons. Du troisième au cinquième jour de phase fébrile, peut apparaître une éruption maculo-papuleuse congestive durant trois à quatre jours (dengue classique).

Dans sa forme sévère, l'infection peut aboutir à l'apparition d'un syndrome hémorragique (DHF ou *dengue haemorrhagic fever*), caractérisé par une perméabilité vasculaire accrue et un dérèglement de l'hémostase. Alors que dans la majorité des cas, l'évolution est généralement favorable en une semaine, l'issue de la maladie peut être fatale en cas de choc hypovolémique (DSS ou *dengue shock syndrome*). Ces complications pourraient être dues à la présence d'une immunité préexistante, acquise notamment lors d'une primo-infection par un virus hétérologue de la dengue (sérotype différent). En effet, deux types différents de réponse sérologique sont identifiés chez les sujets infectés par la dengue : les individus qui n'ont jamais eu une infection à flavivirus et n'ont pas été vaccinés contre un autre flavivirus (virus de la fièvre jaune, virus de l'encéphalite japonaise par exemple) vont présenter une réponse primaire, caractérisée par une apparition lente des anticorps spécifiques du virus responsable de l'infection : les individus qui ont déjà eu une infection à flavivirus (autre sérotype de la dengue, par exemple) ou ont été vaccinés contre un autre flavivirus vont présenter une réponse secondaire, caractérisée par une apparition rapide des anticorps.

L'agent infectieux est le virus de la dengue appartenant à la famille des *Flaviviridae,* à laquelle appartiennent également le virus de la fièvre jaune et celui de l'encéphalite japonaise (T.P. Monath et al., (1996) Flaviviruses in B.N. Fields, D.M. Knipe, P.M. Howly et al. (eds.) "Fields Virology " Philadelphia : Lippincott Raven Press Publishers). Ces virus possèdent un ARN monocaténaire de polarité positive qui comprend 11000 nucléotides et qui code pour une polyprotéine d'environ 3400 acides aminés. Elle s'individualise en trois protéines de structure et sept protéines non structurales NS1, NS2A, NS2B, NS3, NS4A, NS4B et NS5, au cours de clivages co- et post-traductionnels par des protéases virales et cellulaires. La protéine non structurale NS1 a été identifiée pour la première fois en 1970 par P.K. Russel et al. *(J. immunol.,* (1970), **105**, 838-845) et caractérisée en 1985 par G.W. Smith et al. *(J. Gen Virol.,* (1985), **66**, 559-571). Cette glycoprotéine hautement conservée dans le genre des flavivirus (T.P. Monath déjà cité), notamment dans les quatre sérotypes du virus de la dengue, existe sous une forme intracellulaire, et sous une forme extracellulaire. La forme intracellulaire interviendrait dans les phases précoces de la réplication du virus (Hall R. A. et al., *J. Virol.* (1999), **73**, 10272-10280 ; Rice C. M. et al. , *J. Virol.,* (1997), **71**, 291-298 ; Rice C. M. et al., *J. Virol.,* (1996), **222**, 159-168 ; Rice C. M. et al., *J. Virol.,* (1997), **71**, 9608-9617). Avant d'être transportée vers la membrane plasmique, la protéine NS1 subit une dimérisation. Dans les cellules de mammifères, mais pas dans les cellules d'insectes, une partie de la protéine NS1 est libérée dans le milieu extracellulaire soit majoritairement sous forme d'une protéine soluble, soit accessoirement sous forme microparticulaire. Lorsqu'elle est sous forme soluble, la protéine existe sous forme d'un oligomère, notamment d'un pentamère ou d'un hexamère (Crooks A.J. *et al. J Chrom.* (1990), **502**, 59-68 et *J. Gen. Virol.* (1994), **75**, 3453-3460). A l'heure actuelle la fonction biologique de la protéine NS1 n'est pas connue.

Plusieurs études suggèrent un caractère immunodominant de la protéine NS 1 dans la réponse immunitaire protectrice contre les infections à flavivirus. Des expériences réalisées avec un certain nombre de flavivirus tels que les virus de la fièvre jaune, de la dengue, de l'encéphalite japonaise et de l'encéphalite à tique ont montré une protection partielle ou totale contre une dose mortelle de virus homologue chez les animaux vaccinés à l'aide de la protéine NS1 sous-unitaire ou produite par des virus vecteurs, type vaccine ou adénovirus (Schlesinger *et al.,* J. Virol ( 1986), **60**, 1153-1135 ;. *J. Gen. Virol.,* (1987), **68,** 853-857; Falgout *et al., J Virol.,* (1990), **64,** 4356-4363 ; Jacobs *et al., J Virol.,* (1992), **66,** 2086-2095; Hall *et al., J Gen. Virol,* (1996), **77**, I287-1294 ; Konishi *et al., Virology,* (1991), **185**, 401-410).

L'immunisation passive de souris par des anticorps monoclonaux anti-NS1 a également permis d'obtenir un certain degré de protection (Schlesinger et *al., J Immunol.* (1985), **135**, 2805-2809 ; Gould *et al., J. Gen. Virol.,* (1986), 67, 591-595; Henchal *et al., J. Gen. Virol.,* (1988), **69,** 2101-2107). On ne connaît pas totalement le rôle des anticorps anti-NS1 dans la protection. Il se pourrait que les protéines NS1 à la surface des cellules infectées soient reconnues par les anticorps fixant le complément conduisant à la lyse des cellules infectées, (Schlesinger *et al., Virology,* (1993), **192**, 132-141.

Il n'existe pas de traitement spécifique et les soins apportés au patient sont uniquement symptomatiques. Dans le cas de la dengue classique, le traitement repose sur l'administration d'antalgiques et d'antipyrétiques. Dans le cas de DHF, le traitement consiste en une perfusion pour compenser la fuite plasmatique, associée à la correction des troubles hydroélectriques et la relance de la diurèse.

Il n'existe pas de vaccin commercialisé contre le virus de la dengue. En revanche des essais de protection par des souches atténuées des 4 sérotypes du virus de la dengue ont été effectués par N. Bhamarapravati *et al.(Dengue and Dengue haemorrhagic fever* (1997), 367-377) avec des résultats non satisfaisants. La prévention repose donc uniquement sur la lutte contre le vecteur. Cette lutte associe une destruction larvaire et des pulvérisations '' adulticides ".

En l'absence de vaccin, il est nécessaire de surveiller les épidémies et prévenir les complications précitées ; pour ce faire, des programmes de surveillance active ont notamment été mis en place par l'Organisation Mondiale de la Santé et comprennent essentiellement la surveillance des cas de fièvre et des insectes vecteurs et le dépistage sérologique et virologique des personnes présentant une fièvre et suspectées être infectées par le virus de la dengue.

L'étiologie de la dengue est parfois délicate à affirmer lorsqu'un malade présente un syndrome fébrile indifférencié type " dengue-like " qui peut avoir comme origine un autre arbovirus, des virus provoquant des fièvres éruptives telle que la grippe ou des pathogènes non-viraux agents de maladies telles que la leptospirose et même le paludisme. Seul un examen de laboratoire peut apporter le diagnostic.

Il existe à l'heure actuelle plusieurs tests de diagnostic de la dengue. Toutefois, pour arriver à un résultat interprétable, il est nécessaire de combiner plusieurs méthodes :
- l'isolement du virus, par des techniques de virologie classique, notamment par infection de cultures de cellules ou propagation en cerveau de souriceaux ou amplification par inoculation aux moustiques et examen par exemple par immunofluorescence. Ces méthodes ont l'inconvénient d'être difficiles à mettre en oeuvre et de dépendre de la précocité du prélèvement et de bonnes conditions de conservation ; de plus, les premiers résultats ne peuvent être obtenus en moins d'une semaine ; pour pallier à ces inconvénients, on peut avoir recours à un test par RT/PCR (V. Deubel, *L'eurobiologiste* (1997), tome XXXI, 37-155) ; toutefois, ce moyen n'est pas toujours fiable, et ne peut pas être utilisé en routine dans les pays concernés par l'infection par le virus de la dengue pour des raisons de coût et d'équipement ;
- les tests sérologiques ; le diagnostic sérologique le plus précoce consiste à rechercher des IgM spécifiques des antigènes viraux par la technique MAC-ELISA (*immunoglobulin M Antibody Capture Enzyme-Linked ImmunoSorbent Assay*). Leur détection plusieurs jours après le début des symptômes permet d'établir un diagnostic de probabilité d'infection par un flavivirus. Les anticorps de type IgG apparaissent plus tardivement que les anticorps de type IgM. Dans tous les cas, la recherche des anticorps nécessite deux prélèvements : l'un au début des signes cliniques, l'autre 10 à 28 jours après, de façon à mettre en évidence une conversion sérologique par réaction d'inhibition de l'hémagglutination (IHA) ou par ELISA.

Il a également été proposé des tests immunologiques simples et peu chers, utilisables dans les pays à risque, qui mettent en oeuvre, comme réactif immunologique spécifique des peptides dérivés de la protéine non structurale NS1 caractéristique des flavivirus. Ainsi, le brevet US 5 824 506 décrit une méthode utilisant des peptides dérivés de la protéine non structurale NS1, qui permet de détecter les anticorps induits par la présence du virus de la dengue ; toutefois les peptides sélectionnés reconnaissent essentiellement les échantillons obtenus à partir de sujets convalescents et reconnaissent également mieux les patients infectés pour la seconde fois que ceux infectés pour la première fois ; ces résultats décevants pourraient s'expliquer par le fait que les peptides utilisés ne sont pas représentatifs des caractéristiques antigéniques de la protéine native et donc conduisent à une mauvaise reconnaissance des anticorps recherchés.

Dans tous les cas, seule la confirmation tardive d'une infection par un flavivirus peut être apportée.

Un rapport émanant du *Sir Albert Sakzewski Virus Research Centre Royal Children's Hospital,* (A. Falconar, 1991) décrit la recherche de la glycoprotéine non structurale NS1 dans le sérum de patients infectés par le virus DEN2. Les auteurs de ce rapport ont mis au point un test ELISA de type double sandwich, dans lequel un sérum de lapin contenant des anticorps polyclonaux anti-NS1, utilisé comme anticorps de capture, est immobilisé sur une plaque de microtitration. L'antigène capturé est détecté à l'aide d'anticorps monoclonaux de souris dirigés contre la protéine NS1 soit du virus de la dengue de type DEN2, soit spécifique du complexe sérologique de la dengue;. la révélation de la formation du complexe antigène/anticorps est effectuée à l'aide d'IgG de chèvre anti-souris conjuguées à la peroxydase. Par cette méthode, les Auteurs ont montré qu'en utilisant de la protéine NS1 dimérique purifiée ou dégradée comme standard, la sensibilité de détection du test est d'environ 4 ng/ml avec les anticorps monoclonaux DEN2 comme sonde de révélation et d'environ 60 ng/ml avec les anticorps monoclonaux de groupe.

Toutefois, ce test ne permet de détecter la protéine NS1 ni dans les cas d'infections primaires en phase aiguë ou convalescente, ni dans les infections secondaires en phase convalescente dans lesquelles il existe un titre élevé en anticorps anti-NS1 ; les Auteurs en ont conclu que la protéine NS1 devait être présente en quantités importantes uniquement dans les cas d'infections secondaires et ce, de manière transitoire, pendant l'infection.

Or, les Inventeurs ont mis au point un procédé de purification de la protéine NS1 d'un flavivirus sous forme hexamérique, ce qui leur a permis de sélectionner des anticorps spécifiques de cette protéine sous forme hexamérique, et de montrer de manière surprenante que ces anticorps sont des outils de choix pour la mise en évidence des différentes formes de la protéine NS1 circulante dans le cadre d'une infection par un flavivirus, en particulier dans les phases précoces où la réponse en anticorps spécifiques est indétectable, notamment lors des infections primaires par le virus de la dengue.

En conséquence, les Inventeurs se sont donné pour but de pourvoir à une méthode de détection précoce d'une infection flavivirale, qui répond mieux aux besoins de la pratique que les procédés de l'état antérieur de la technique, c'est-à-dire un procédé qui soit fiable, rapide, peu coûteux et qui permet d'adapter à temps les soins médicaux.

La présente invention a en conséquence pour objet une méthode de détection précoce d'une infection flavivirale, caractérisée en ce qu'elle comprend la détection de la glycoprotéine non structurale NS1 d'un flavivirus dans un échantillon biologique, pendant toute la durée de la phase clinique de l'infection, par une méthode immunologique mettant en oeuvre au moins deux anticorps identiques ou différents,
- le premier anticorps ou anticorps de capture de la glycoprotéine NS1 étant constitué par des anticorps choisis dans le groupe constitué par :
   - des anticorps polyclonaux préalablement sélectionnés par immunocapture sur la protéine NS 1 dudit flavivirus, de forme hexamérique, et
   - des mélanges d'anticorps monoclonaux anti-NS1 préalablement sélectionnés pour leur affinité élevée pour la protéine NS 1 dudit flavivirus, de forme hexamérique, lesdits anticorps monoclonaux étant ensuite purifiés,
- le deuxième anticorps ou anticorps de révélation étant choisi dans le groupe constitué par :

- des anticorps polyclonaux dirigés contre la protéine NS1 de forme hexamérique, et
- un mélange d'anticorps monoclonaux dirigés contre la protéine NS1 de forme hexamérique.

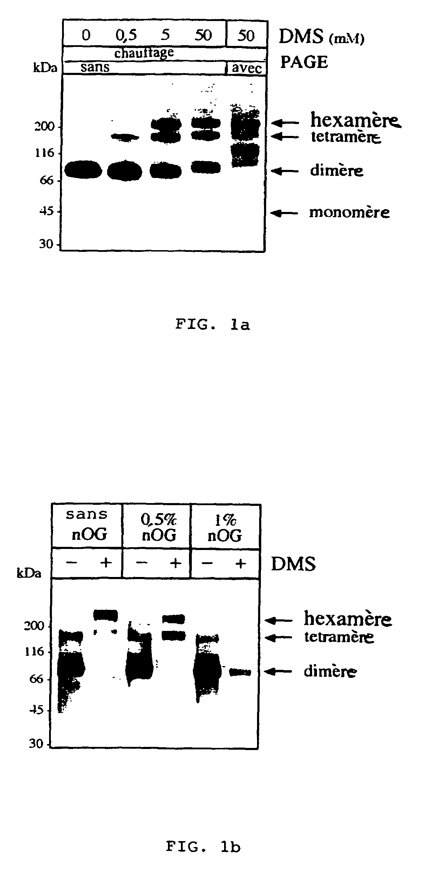

Au sens de la présente invention, on entend par forme hexamérique de la protéine NSI d'un flavivirus, la protéine native obtenue à partir du surnageant de culture de cellules de mammifères infectées par ledit flavivirus ou bien transformées par un système d'expression comportant le gène de la protéine NSI dudit flavivirus, et purifiée selon le procédé de l'invention tel que décrit ci-après. Cette forme hexamérique de ladite protéine NS1 qui se distingue d'autres formes telles que la forme monomérique ou la forme dimérique de ladite protéine, est mise en évidence par des techniques d'électrophorèse ou de chromatographie telles que celles décrites à la figure 1.

Au sens de la présente invention, on entend par anticorps polyclonaux et monoclonaux dirigés contre la protéine NS1 d'un flavivirus, des anticorps obtenus par immunisation d'un mammifère non humain,
- soit par une protéine NS 1 de forme hexamérique,
- soit par un flavivirus vivant ou inactivé,
lesdits anticorps polyclonaux étant sélectionnés pour leur affinité pour la protéine NS 1 sous forme hexamérique et purifiés en une seule étape et lesdits anticorps monoclonaux étant préalablement sélectionnés pour leur affinité élevée pour la protéine NS1 sous forme hexamérique puis purifiés par des techniques classiques, notamment par chromatographie d'affinité ou échange d'ions.

Au sens de la présente invention on entend par affinité d'un anticorps monoclonal pour la protéine NS1 sous forme hexamérique la concentration en ladite protéine nécessaire pour saturer 50 % des sites de l'anticorps, celle-ci est mesurée par la constante d'affinité dudit anticorps selon le protocole décrit à l'exemple 5.

Au sens de la présente invention on entend par affinité élevée une affinité dont la constante est inférieure à 10⁻⁸ M

De manière surprenante, l'utilisation, pour la détection de la protéine NS1 dans un échantillon biologique, d'anticorps polyclonaux sélectionnés et purifiés par immunocapture sur la protéine NS1 de forme hexamérique, ou d'anticorps monoclonaux présentant une affinité élevée pour la protéine NS1 de forme hexamérique et purifiés, en lieu et place d'un sérum total de lapin hyperimmunisé, permet d'améliorer de manière significative la sensibilité de la méthode et de mettre en évidence la protéine NS1 circulant dans le sang des malades, dès le stade précoce de l'infection, aussi bien lors d'une infection primaire que d'une infection secondaire.

La méthode selon la présente invention possède un certain nombre d'avantages:
- elle peut être mise en oeuvre de manière précoce : la présence de la glycoprotéine NS1 est révélée lors de la phase clinique, avant que la réponse en anticorps ne soit détectable,
- elle est sensible : on peut détecter jusqu'à moins de 1 ng de protéine/ml de sérum, ce qui permet de détecter la protéine NS1 circulante dans la phase précoce des infections primaires,
- elle est rapide : on peut obtenir une réponse dans la journée,
- elle est relativement peu coûteuse et peut donc être utilisée dans les pays à risque,
- elle permet de distinguer les personnes vaccinées-des personnes infectées récemment par un flavivirus puisque la protéine NS1 sera absente chez les personnes vaccinées chez lesquelles les anticorps pourraient encore être décelables.

Selon un mode de mise en oeuvre avantageux de ladite méthode l'infection flavivirale est une infection par le virus de la dengue.

Selon un autre mode de mise en oeuvre avantageux de ladite méthode, le premier anticorps est de préférence fixé sur un support solide convenable et le deuxième anticorps est éventuellement conjugué à un marqueur approprié.

Selon un autre mode de mise en oeuvre avantageux de ladite méthode, lorsque le deuxième anticorps n'est pas conjugué à un marqueur, sa liaison à la protéine NS1 fixée sur le support solide est alors détectée par un troisième anticorps conjugué à un marqueur convenable, ledit troisième anticorps étant un anticorps classiquement utilisé, tel que par exemple une IgG dirigée contre le deuxième anticorps et produite notamment chez la chèvre, le porc ou l'âne.

Parmi les marqueurs utilisés, on peut citer à titre d'exemple les marqueurs fluorescents, le système biotine/streptavidine, les marqueurs non isotopiques ou des enzymes, comme par exemple la peroxydase de raifort ou la phosphatase alcaline.

Selon un autre mode de mise en oeuvre avantageux de ladite méthode, ledit troisième anticorps est conjugué à une enzyme.

Selon un autre mode de mise en oeuvre avantageux de ladite méthode,
- le premier anticorps ou anticorps de capture est constitué par des anticorps polyclonaux de souris, sélectionnés par immunocapture sur la protéine NS1 du virus de la dengue, ladite protéine étant sous forme hexamérique, et
- le deuxième anticorps ou anticorps de détection de la présence de NS1 dans l'échantillon biologique à analyser, est constitué par des anticorps polyclonaux de lapin immunisé par de la protéine NS1 du virus de la dengue, ladite protéine étant sous forme hexamérique, la fixation dudit deuxième anticorps étant révélé par un troisième anticorps constitué par des anticorps, conjugués à la peroxydase et dirigés contre le deuxième anticorps.

Selon un autre mode de mise en oeuvre encore plus avantageux de ladite méthode, les anticorps polyclonaux de souris sont purifiés par immunocapture sur de la protéine NS 1 hexamérique de dengue de sérotype 1.

La présente invention a également pour objet un kit ou coffret de diagnostic d'une infection flavivirale, caractérisé en ce qu'il comprend :
- au moins un anticorps de capture et au moins un anticorps de révélation tels que définis précédemment,
- au moins un contrôle positif constitué par la protéine NS1 d'un flavivirus et/ou de différents sérotypes selon le flavivirus, ladite protéine, étant sous forme hexamérique, et
- au moins un contrôle négatif constitué par un sérum humain normal.

Selon un mode de réalisation avantageux du coffret de diagnostic selon l'invention, ladite protéine NS1 sous forme hexamérique est obtenue à partir d'un surnageant de culture, soit de cellules de mammifères infectées, soit de cellules de mammifères transfectées par un plasmide recombiné, comportant le gène de la protéine NS1 ou un fragment dudit gène ou un fragment du génome flaviviral, lesdits fragments étant aptes à exprimer tout ou partie de la protéine NS 1.

Selon un autre mode de réalisation avantageux du coffret de diagnostic selon l'invention, la protéine NS 1 est celle du virus de la dengue.

Selon un autre mode de réalisation encore plus avantageux dudit coffret de diagnostic, le plasmide est le plasmide pClneo-NS1.FGA qui a été déposé auprès de la Collection Nationale de Cultures et de Microorganismes tenue par l'Institut Pasteur sous le numéro I-2220, en date du 7 juin 1999.

La présente invention a également pour objet un procédé de purification de la protéine NS 1 d'un flavivirus, sous forme hexamérique, à partir d'un surnageant de culture, soit de cellules de mammifères infectées, soit de cellules de mammifères transfectées par un plasmide recombiné, comportant le gène de la protéine NS1 d'un flavivirus ou un fragment dudit gène ou un fragment du génome flaviviral, lesdits fragments étant aptes à exprimer la protéine NS1 sous forme hexamérique, caractérisé en ce que préalablement à la purification de la protéine NS1 par des techniques classiques telles que la chromatographie d'affinité, l'on sépare la forme soluble de la protéine NS1 de la forme microparticulaire de ladite protéine, par traitement par un agent précipitant puis par centrifugation.

Par exemple, la centrifugation est réalisée à une vitesse supérieure ou égale à 10 000 g.

Au sens de la présente invention, on entend par agent précipitant, un agent qui précipite spécifiquement les protéines microparticulaires ou débris cellulaires, comme par exemple le polyéthylèneglycol, ledit agent étant utilisé dans des conditions classiques qui permettent de séparer des protéines solubles et des protéines microparticulaires ou débris cellulaires.

Dans un mode préféré de mise en oeuvre dudit procédé de purification, la protéine hexamérique NS1 est celle du virus de la dengue, notamment le virus de la dengue de sérotype 1.

La présente invention a également pour objet une composition immunogène, caractérisée en ce qu'elle comprend à titre de principe actif la protéine NS1 d'un flavivirus, de forme hexamérique, éventuellement associée à d'autres protéines, en association avec au moins un véhicule pharmaceutiquement acceptable.

Dans un mode préféré de réalisation de la composition immunogène selon la présente invention, la composition immunogène comprend au moins un mélange des protéines NS1 de forme hexamérique correspondant aux différents sérotypes du virus de la dengue.

La présente invention a également pour objet une composition immunogène, caractérisée en ce qu'elle comprend un principe actif sélectionné dans le groupe constitué par :
- un polynucléotide capable d'exprimer tout ou partie de la protéine NS 1 du virus de la dengue quel que soit son sérotype,
- un système d'expression comprenant au moins un promoteur capable de faire exprimer chez l'hôte où il est injecté, un ADN codant pour la protéine NS1 du virus de la dengue quel que soit son sérotype, ledit ADN exprimant ladite protéine,
en association avec au moins un véhicule pharmaceutiquement acceptable.

Des protocoles de vaccination utilisant des acides nucléiques sont notamment décrit dans la demande internationale WO 90/11092.

La présente invention a également pour objet l'utilisation d'une protéine NS1 de forme hexamérique d'un flavivirus ou d'un de ses systèmes d'expression, pour la préparation d'une composition immunogène capable d'induire la production d'anticorps *in vivo.*

Dans un mode préféré de ladite utilisation, la protéine NS1 est celle du virus de la dengue, notamment de sérotype 1.

La présente invention a également pour objet l'utilisation d'au moins un anticorps monoclonal anti-NS 1 présentant une affinité élevée pour la protéine NS1 de forme hexamérique, lesdits anticorps monoclonaux étant ensuite purifiés, et modifiés pour la fabrication d'un médicament apte à induire une immunisation passive.

De manière avantageuse les modifications des anticorps sont notamment la sélection de fragments Fab ou l'humanisation des anticorps.

La présente invention a également pour objet l'utilisation de la protéine NS1 de forme hexamérique, pour sélectionner *in vitro* des anticorps spécifiques de ladite protéine NS1 aptes au diagnostic précoce d'une infection par un flavivirus.

Dans un mode de réalisation avantageux de ladite utilisation, les anticorps sont des anticorps polyclonaux.

Dans un autre mode de réalisation avantageux de ladite utilisation, les anticorps sont des anticorps monoclonaux.

Dans un autre mode de réalisation avantageux de ladite utilisation, la protéine est la protéine NS 1 du virus de la dengue, notamment de sérotype 1.

Les anticorps monoclonaux anti-NS1 sont avantageusement obtenus par fusion de cellules spléniques de souris immunisée par la protéine NS1 de forme hexamérique avec des cellules myélomateuses appropriées.

La présente invention a également pour objet un procédé d'expression d'un polynucléotide codant pour la protéine NS1 d'un virus de la dengue, caractérisé en ce qu'il comprend l'expression d'un polynucléotide tel que défini à la séquence SEQ ID N° 1, associé à un promoteur dudit polynucléotide dans des cellules eucaryotes convenables.

D'autres caractéristiques et avantages de l'invention apparaissent dans la suite de la description et des exemples illustrés par les figures dans lesquelles :
- la figure 1 représente la protéine NS1 extracellulaire hexamérique purifiée obtenue après chromatographie d'exclusion (a) après chromatographie d'exclusion, la protéine est concentrée à 0,5 mg/ml par ultrafiltration et traitée par du diméthylsuberimidate (DMS) à 0, 0,5, 5 et 50 mM. Les produit obtenus sont placés dans un tampon non réducteur de Laemmli, séparés sur un gel d'acrylamide de gradient 4 à 20 % et marqués au bleu de Coomassie. Un échantillon traité par du DMS 50 mM est chauffé pendant 3 min à 95 °C avant électrophorèse pour dissocier les oligomères non covalents. (b) La protéine NS1 purifiée est traitée pendant une nuit à 37 °C par 0,5 % ou 1 % de *n*-octylglucoside (nOG) et éventuellement traitée par 25 mM de DMS pendant 1 heure. Les protéines sont séparées sans dénaturation à chaud sur un gel d'acrylamide de gradient 4 à 20 % et détectée par *immunoblotting* avec un anticorps monoclonal anti-NS1 de la littérature ou tel que défini précédemment.

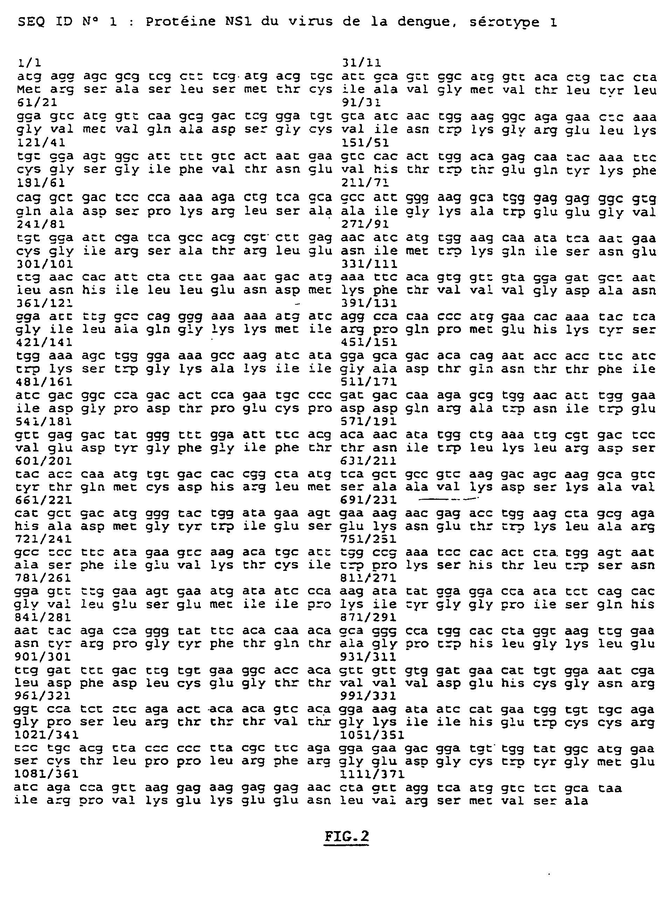

- la figure 2 représente la séquence de la protéine NS 1 du virus de la dengue de sérotype 1 obtenue par le clone 4C de l'exemple 2 ci-après, ainsi que la séquence codante correspondante.

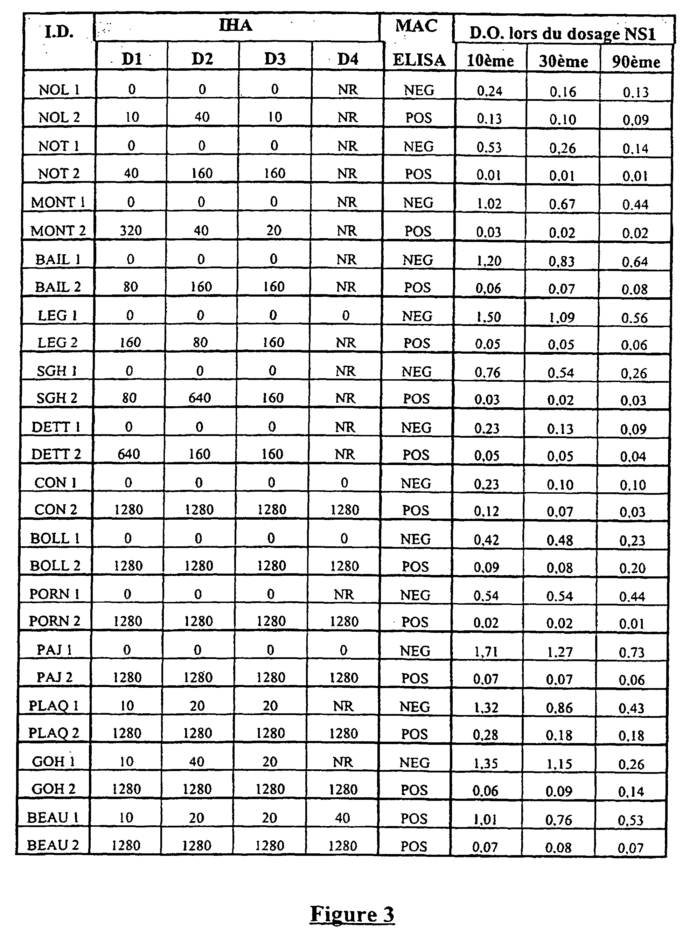

- la figure 3 illustre les résultats obtenus par dosage de la protéine NS1 circulante par la méthode de détection par ELISA-capture chez des patients infectés préalablement par un virus de la dengue dont les sérums ont été prélevés lors des phases aiguës et convalescentes, ainsi que la comparaison avec les résultats obtenus par les techniques de l'art antérieur IHA (inhibition d'hémagglutination des sérotypes 1, 2, 3 ou 4 du virus de la dengue) et .MAC ELISA *(immunoglobulin M Antibody Capture Enzyme-Linked ImmunoSorbent Assay*) ; D1 correspond au sérotype 1 de la dengue ; D2 correspond au sérotype 2 de la dengue; D3 correspond au sérotype 3 de la dengue et D4 correspond au sérotype 4 de la dengue ; ID = identité du malade ; 1 correspond au premier prélèvement en phase aiguë de la maladie 2 correspond au deuxième prélèvement en phase convalescente (effectuée 2 à 4 semaines après le premier) ; dans le test ELISA-capture, les valeurs sont exprimées en densité optique obtenues pour un même sérum dilué 10, 30 ou 90 fois.

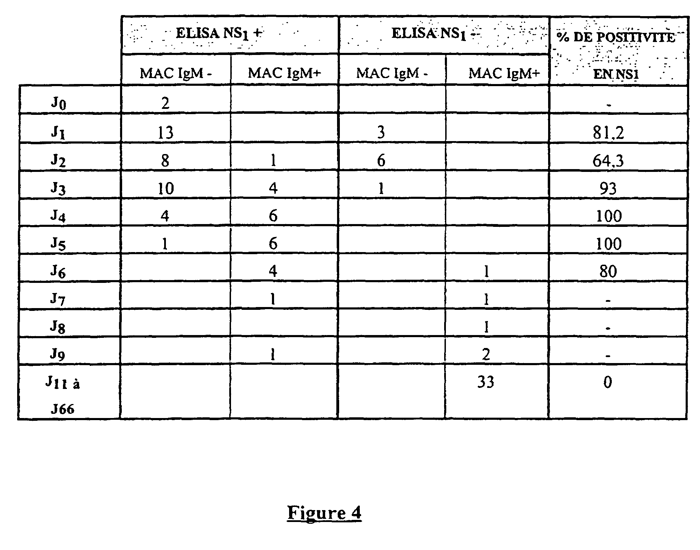

- la figure 4 illustre la détection de la protéine NS1 par le test ELISA-capture sur des sérums de patients infectés par le virus de la dengue de sérotype 1 de Guyane française. Les chiffres indiqués représentent le nombre de patients répartis par catégorie (positivité ou négativité en ELISA-capture et positivité ou négativité en IgM).

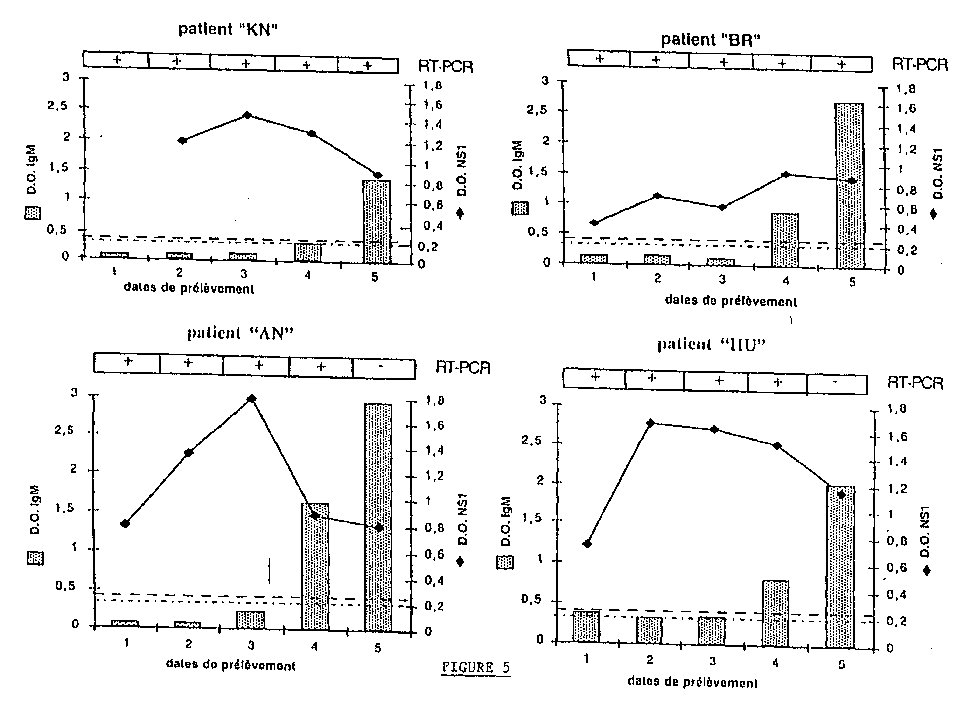

- la figure 5 illustre les résultats obtenus pour 4 patients de Guyane française infectés par le virus dengue 1 qui ont été prélevés quotidiennement pendant la phase clinique de la maladie de J1 à J5. Chaque graphe correspond à un patient avec pour chaque jour de prélèvement, à la fois les résultats de la détection de la protéine NS1 avec le test ELISA-capture développé, les résultats de RT-PCR et les résultats obtenus par la technique MAC-ELISA. Les valeurs de D.O. rapportées ont été corrigées d'une fois la valeur du bruit de fond. Les seuils de positivité sont indiqués par les traits en pointillé.

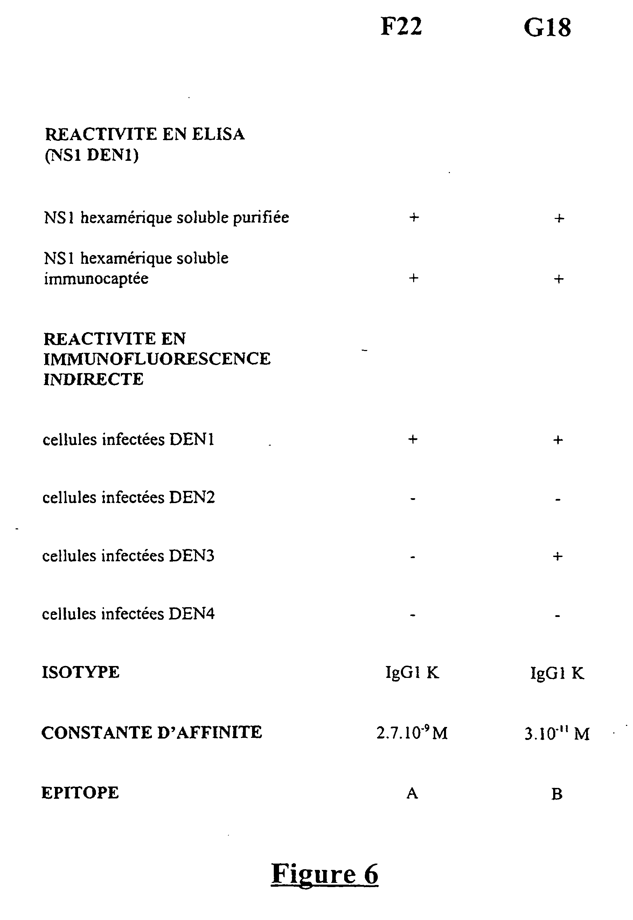

- la figure 6 indique les caractéristiques des anticorps monoclonaux anti NS1 F22 et G18

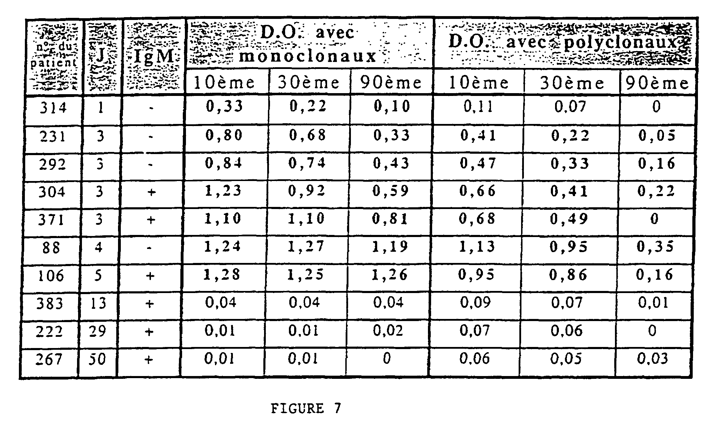

- la figure 7 illustre la détection de la protéine NS1 par le test ELISA-capture utilisant l'approche monoclonale par comparaison avec le test ELISA-capture utilisant l'approche polyclonale telle que décrite dans l'exemple 3. Les résultats obtenus sont rapportés sous la forme des valeurs de densité optique mesurées pour chaque dilution de sérum analysé (10ème, 30ème ou 90ème) et retranchées de la valeur moyenne des témoins négatifs.

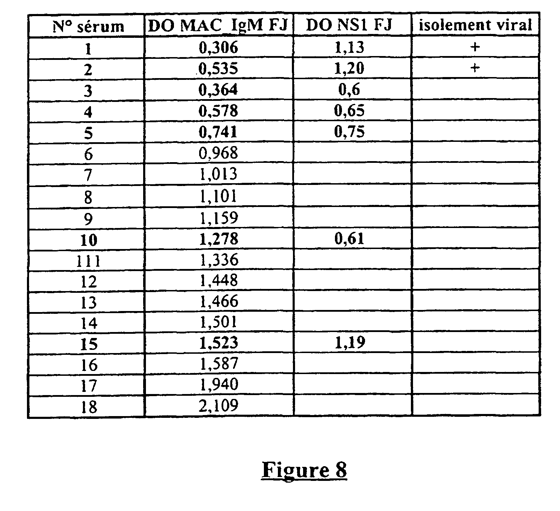

- la figure 8 illustre la mise en évidence de la protéine NS1 dans les sérums de patients infectés par le virus de la fièvre jaune, par un test ELISA-capture spécifique de la fièvre jaune. Pour chaque sérum testé est reportée la valeur de densité optique mesurée par le test développé retranchée de la valeur moyenne des témoins négatifs, la valeur de densité optique mesurée par le test MAC-ELISA spécifique des IgM de fièvre jaune et les résultats de l'isolement viral lorsqu'ils sont disponibles.

### Exemple 1 : Purification de la protéine NS1 du virus de la dengue, sérotype 1

### 1. Matériel et méthodes

La protéine est produite sur cellules Véro infectées par le virus de la dengue, sérotype 1, souche FGA/89 (P. Després *et al., Virol,* (1993), **196**, 209-219), dans les conditions adaptées de la méthode décrite par A.K.I. Falconar *et al.,* (*J. Virol. Meth.,* (1990), **30**, 323-332).

Le milieu de culture est récolté 5 jours après l'infection et centrifugé à 1500 g pour éliminer les débris cellulaires. Le surnageant de centrifugation est ramené à 20 mM Tris-HCl, 1 mM azide de sodium, concentré 6 fois par ultrafiltration à froid et les particules virales sont éliminées par une précipitation de 2 h à 4 °C, à une concentration de 7,5 % en polyéthylèneglycol (PEG), suivie d'une centrifugation de 30 min. à 10 000 g. Le surnageant clarifié, contenant 7,5 % PEG est traité avec 0,05 % Tween 20 et 1 mg/ml d'aprotinine, puis passé sur une colonne d'immunoaffinité sur laquelle est fixé un anticorps monoclonal anti-NS1. La protéine est éluée selon la technique à la diéthylamine, telle que décrite dans Falconar *et al.,* (précité), concentrée par ultrafiltration et la solution d'élution échangée par un tampon 10 mM Tris-HCl pH 7,5 contenant 1 mM d'azide de sodium.

### 2. Résultats

Les résultats sont illustrés dans la figure 1.

La figure 1a montre que la protéine NSI est bien sous forme hexamérique. La proportion de forme hexamérique augmente avec une concentration croissante en DMS (figure 1a).

La protéine de forme hexamérique extracellulaire NSI peut être transformée en sous-unités dimériques en présence du détergent non ionique, *n*-octylglucoside (nOG) (figure 1b). Après une nuit à 37°C en absence ou en présence de n-octylglucoside (nOG) et traitement par du DMS 25 mM, on observe, en l'absence de nOG, la présence de bandes correspondant au dimère, au tétramère et à l'hexamère et en présence de nOG, une dissociation partielle ou complète de l'hexamère en fonction de la concentratin en nOG (figure 1b).

### Exemple 2: Expression de la protéine NS1 du virus de la dengue de sérotype 1 par des cellules Vero

### 1. Matériel et méthodes

Le plasmide pCIneo-NS1.FGA déposé auprès de la Collection Nationale de Cultures et de Microorganismes (CNCM) de l'Institut Pasteur sous le n° 1-2220, en date du 7 juin 1999) contenant le gène de la protéine NS1 comprenant le gène codant pour son peptide signal, précédé d'un codon d'initiation de la traduction et suivi d'un codon de terminaison de la traduction est introduit dans la bactérie compétente *Escherichia coli* (Epicurian SURE de Stratagène). Ce plasmide est amplifié en culture bactérienne et purifié selon la technique classique de préparation d'ADN plasmidique. L'ADN purifié est utilisé pour le séquençage de différents clones (la séquence du clone 4C est illustrée à la figure 2) et pour transfecter des cellules Véro à l'aide soit d'un mélange adéquat avec des liposomes cationiques, tel que le DOTAP (Boehringer-Mannheim), ou avec un agent non liposomal, tel que le FuGENE (Boehringer-Mannheim). Le FuGENE et l'ADN sont préincubés dans du milieu sans sérum pendant 15 min puis le mélange est mis en contact avec un tapis cellulaire de cellules Vero pendant 24 h. Les cellules sont ensuite rincées à l'aide de PBS (*phosphate saline buffer*), fixées 20 min à température ambiante avec une solution de PBS contenant 3% de paraformaldéhyde, perméabilisées 5 min avec du PBS contenant 0,5% Triton X-100. La présence d'antigène NS1 est alors révélée à l'aide d'anticorps spécifiques, reconnus par un anticorps conjugué marqué à la fluorescéine.

### 2. Résultats

Il est possible de mettre ainsi en évidence par un signal fluorescent élevé, spécifique de l'antigène viral NS1, dans environ 20% des cellules transfectées.

L'expression de NS1 est ainsi démontrée et des lignées stables pourront être établies en présence de néomycine, marqueur de sélection des cellules transfectées.

La figure 2 illustre la séquence de la protéine NS 1 du virus de la dengue de sérotype 1 ainsi obtenue ainsi que la séquence codante correspondante.

### Exemple 3 : Mise en oeuvre de la technique ELISA-capture selon l'invention dans le cadre d'une infection par le virus de la dengue de sérotype 1 et comparaison avec les méthodes de l'état antérieur de la technique

### 1.Principe de la technique ELISA-capture

L'antigène viral NS1 est capturé par des anticorps polyclonaux de souris monospécifiques préalablement purifiés par immunocapture sur la protéine NS 1 hexamérique purifiée de dengue, sérotype 1.

La présence de NS 1 est révélée par des anticorps de lapin immunisés par la protéine NS1 hexamérique purifiée, eux-mêmes reconnus par des anticorps conjugués à la peroxydase de raifort.

### 2. Matériel et méthodes

### a- Purification d'anticorps polyclonaux de souris dirigés contre la protéine NS1

### a₁- Fixation de la protéine NS1 sur membrane

La protéine NS 1 purifiée est fixée par adsorption sur une membrane de nylon amphotérique (Nytran, Schleicher & Schuell). La surface de la membrane est alors saturée par de l'albumine bovine présente à une concentration de 3 % dans une solution saline tamponnée par du phosphate (PBS : *phosphate buffer saline* ; 10 mM phosphate ; pH7,2 ; 150 mM NaCl). Après 2 rinçages dans du PBS, la membrane est traitée par du PBS contenant 0,25 % de glutaraldéhyde pendant 15 min. à température ambiante. Après 3 rinçages dans du PBS, la membrane est neutralisée par un tampon de 100 mM glycine, 3 % albumine bovine, rincée 2 fois dans du PBS puis conservée à 4°C dans du PBS avec de l'azide de sodium 1 mM.

### a₂- Purification des anticorps polyclonaux monospécifiques de souris, (anticorps de capture)

- production des ascites polyclonales: les cerveaux de souriceaux Swiss infectés par le virus de la dengue et moribonds sont broyés dans 9 ml de tampon PBS. Le produit est centrifugé 10 min à 10.000 g à 4° C.

La suspension virale est injectée à des souris Swiss, selon le calendrier suivant :
- J0 : 0,5 ml d'antigène en sous-cutané dans la cuisse,
- J3 : 0,4 ml d'antigène et 0,1 ml d'adjuvant complet de Freund par voie intrapéritonéale,
- J25 : 0,5 ml d'antigène par voie intrapéritonéale,
- J26 : 0,5 ml d'ascite de souris TG180, et
- J28 : 0,5 ml d'antigène par voie intrapéritonéale.

Les ascites sont récoltées à J42.

Après recueil des ascites, on laisse le coagulum se former pendant 1 heure à température ambiante puis on réalise une centrifugation pendant au moins 30 minutes à 1500 g. Le surnageant est laissé une nuit à 4°C. Le pH du surnageant est ajusté à 4,8 avec de l'acide acétique 2 M puis le surnageant est centrifugé de nouveau dans les mêmes conditions. Le pH du surnageant est alors amené à_{.} 7,0 - 7,2 par addition d'une solution de soude 2 N. Le surnageant peut être conservé à - 20°C.
- purification des anticorps de souris spécifiques du virus de la dengue de sérotype 1:
   La membrane est incubée pendant une heure à température ambiante dans un mélange d'ascites polyclonales dirigées contre les 4 sérotypes du virus de la dengue préparées comme décrit ci-dessus.
      Après 3 rinçages de la membrane dans du PBS, les anticorps fixés sur la protéine NS 1 sont élués avec une solution de diéthylamine pH 11,4 (milieu Dubelco modifié par Iscove (Gibco) contenant 100 mM diéthylamine). Les anticorps sont concentrés par ultrafiltration et replacés dans un tampon PBS contenant de l'azide de sodium 1 mM.

### b- Préparation d'anticorps polvclonaux de lapin dirigés contre la protéine NS 1 (anticorps de révélation) :

L'immunisation des lapins a été réalisée par 3 ou 4 injections successives de 30 µg de protéine NS1 hexamérique purifiée selon la méthode de l'exemple 1, réalisées à J0 J7, J21 et éventuellement à J49 et suivies d'une saignée à blanc à J83. Le sérum est appauvri en signal non spécifiques par incubation avec des billes de Sépharose portant un anticorps monoclonal décrit dans la littérature ou préparé comme décrit précédemment.

### c- Procédé ELISA-capture

### c₁- Courbe étalon

Pour chaque plaque ELISA-capture destinée à tester des sérums humains, une gamme étalon est réalisée à partir d'une solution de protéine NS1 purifiée selon la méthode décrite au point 1, dont la concentration initiale est de 0,5 µg/ml et diluée de 3 en 3.

### c₂- Détection de la protéine NS1 circulante lors de la phase aiguë :

Les anticorps polyclonaux de souris purifiés obtenus selon la méthode décrite précédemment (anticorps de capture) sont fixés sur une plaque, dilués dans une solution de PBS et mis à incuber pendant une nuit à 4°C. Après 3 rinçages de 5 minutes avec une solution de PBS/Tween 0,05 %, la plaque est saturée avec un mélange de PBS, Tween 0,05 % et de lait 3 %, pendant 30 minutes à température ambiante. Après 3 rinçages avec une solution de PBS / Tween 0,05 %, on dépose les les sérums à tester, dilués ou non, et on laisse réagir pendant une heure, toujours à température ambiante. Les dilutions au 1/10, au 1/30 et au 1/90 sont réalisées dans une solution de PBS / Tween 0,05 %. Après 3 rinçages, on ajoute le second anticorps spécifique de NS1 (anticorps de révélation obtenu au point 3 précédent), après l'avoir dilué dans un mélange de PBS / Tween 0,05 % et de lait 3 %, et on laisse incuber pendant 45 minutes à 37°C. Après 3 rinçages, l'anticorps anti-IgG dirigé contre le deuxième anticorps et marqué à la peroxydase, ledit anticorps étant préparé dans des conditions classiques connues de l'homme du métier, est ajouté et l'incubation réalisée pendant 45 minutes à 37°C. Après 3 rinçages, on révèle pendant 10 minutes par une solution de TMB (3,3', 5,5'-tétraméthylbenzidine, Kierkegaard & Perry Lab). La réaction colorimétrique est arrêtée avec de l'acide sulfurique.

### 3. Résultats

Ils sont illustrés dans la figure 3.

La technique ELISA-capture selon l'invention permet de détecter la présence de la protéine NS 1 dans la phase aiguë de la maladie et ce indépendamment du fait que les malades aient une infection primaire ou secondaire.

Les résultats confirment que la présence de la protéine NS1 est transitoire, puisque dans les prélèvements réalisés en phase de convalescence, on ne détecte pas cette protéine (figure 3).

93 % des prélèvements en phase aiguë de la maladie, se révèlent négatifs par le test MAC ELISA, alors que 100 % des prélèvements réalisés en phase convalescente se révèle positif dans ce même test (figure 3).

De la même façon, le test d'inhibition d'hémagglutination (IHA) ne permet pas de détecter l'infection par le virus de la dengue de sérotype 1 dans 80 % des cas en phase aiguë de la maladie mais ce test se révèle positif dans 100 % des prélèvements réalisés en phase convalescente (figure 3). Selon les critères OMS, un taux d'IHA inférieur à 1280 dans le sérum prélevé en phase convalescente permet de diagnostiquer une infection de dengue primaire et un taux supérieur à 1280 une infection de dengue secondaire. La moitié des sérums positifs de cette étude correspondent donc à des cas de dengue primaire et l'autre moitié à des cas de dengue secondaire. La technique ELISA-capture selon l'invention permet ainsi de détecter de la protéine NS1 dans les cas de dengue primaire et secondaire.

### Exemple 4 : Détermination de la fenêtre de détection

### 1. Matériels et méthodes

### a- étude réalisée sur une cohorte de patients de Guyane française infectés par le virus dengue 1

Les prélèvements sont réalisés chez des patients infectés par le virus de la dengue de sérotype 1, entre J0, marquant l'apparition des signes cliniques (initialement une fièvre indifférenciée) et J66 correspondant à la fin de la phase convalescente.

Sur les sérums de ces patients, on recherche la présence de NS1 circulante selon la méthode ELISA-capture décrite dans l'exemple 3 et on compare le résultat obtenu avec la positivité en IgM spécifiques mesurée en MAC-ELISA, lorsque les données sont disponibles.

### b- suivi quotidien de 4 patients infectés par le virus dengue 1

Quatre patients ont été prélevés quotidiennement pendant la phase clinique de J1 à J5. Une réaction de RT-PCR pour mettre en évidence l'ARN viral, un test MAC-ELISA pour la détection d'IgM spécifiques du virus de la dengue et une recherche de l'antigène NS1 dengue selon la méthode ELISA-capture décrite précédemment, ont été réalisés sur chaque prélèvement sanguin.

### 2. Résultats

### a- Détermination de la fenêtre de détection

Les résultats sont rassemblés dans la figure 4.

Entre J1 et J6, la possibilité de détecter la protéine NS1 circulante oscille entre 64 % (à J2) et 100 % (à J5) des patients infectés. Au delà de J10, la protéine NS1 circulante n'est plus détectée, alors que la réponse en anticorps devient prédominante.

La détection de la protéine NS1 circulante ne semble pas dépendante de la présence d'IgM totales (spécifiques des antigènes viraux) qui apparaissent dans certains cas à J3 et culminent à partir de J5, ni même, pour certains patients de la présence d'IgG totales qui peuvent apparaitre dès J2. En revanche, l'absence de détection de l'antigène NS1 dans des sérums de la phase clinique pourrait s'expliquer par la présence d'IgG spécifiquement dirigées contre NSI.

Ainsi, la fenêtre de détection de l'antigène sérique NS1 par la technique ELISA- capture selon la présente invention se situe de préférence entre J1 et J6 après l'apparition des signes cliniques.

### b- suivi quotidien de 4 patients infectés par le virus dengue

Les résultats sont rassemblés dans la figure 5.
Chez les 4 patients étudiés, la protéine NS1 est détectée de manière continue jusqu'à J5, et ce, quelque soit le jour de prélèvement par rapport au début des symptômes. Pour certains patients, la fenêtre de détection de la protéine est plus large que la période de virémie, détectée par RT-PCR.

### Exemple 5: Mise en oeuvre de la technique ELISA-capture avec des outils monoclonaux dans le cadre d'une infection par le virus de la dengue de sérotype 1 et comparaison avec la technique ELISA-capture précédemment décrite

### 1. Matériel et méthodes

### a - Production et caractérisation d'anticorps monoclonaux de souris dirigés contre la protéine NS1 du virus de la dengue sérotype 1

### a₁- Production d'anticorps monoclonaux de souris dirigés contre la protéine NS1

L'immunisation des souris Balb/C femelles a été réalisée par 7 injections de 10 µg de protéine NS1 du virus de la dengue sérotype 1, hexamérique, purifiée selon la méthode de l'exemple 1. La première injection en adjuvant complet de Freund et les cinq suivantes en adjuvant incomplet de Freund sont réalisées par voie sous-cutanée à 15 jours d'intervalle. La dernière injection, en adjuvant incomplet de Freund, pratiquée trois jours avant le sacrifice de l'animal est effectuée par voie intrapéritonéale.

Les cellules de la rate des souris immunisées sont fusionnées avec le myélome murin et mises en culture jusqu'à l'apparition des clones selon le protocole standard.

### a₂- Identification des hybridomes sécrétant des anticorps anti-NS1

La détection des anticorps spécifiques de la protéine NS1 a été réalisée soit par une technique ELISA classique soit par une technique ELISA-capture

### -Technique ELISA classique

La protéine NS1 hexamérique purifiée selon la méthode de l'exemple 1 est fixée par adsorption sur une plaque à la concentration de 1 µg/ml en solution de PBS pendant une nuit à 4°C. Après 3 lavages avec une solution de PBS/Tween 0,1% (PT) la protéine est incubée avec les surnageants des différents hybridomes dilués au 1/2 avec une solution de PT contenant 0,5% de gélatine (PTG) pendant 1h à 37°C. Après 3 lavages avec PT, l'anticorps anti-IgG de souris marqué à la péroxydase dilué en PTG est ajouté et incubé pendant 1h à 37°C. Après 3 lavages, on révèle par une solution d'eau oxygénée en présence d'orthophénylènediamine.

### - Technique ELISA-capture

La technique utilisée est décrite dans l'exemple 3 (cf Détection de la protéine NS1 circulante dans la phase aiguë) mais en remplaçant les dilutions de sérums à tester par une dilution au 1/10 dans la solution PTG de surnageant de culture de cellules Vero non infectées ou infectées pendant 5 jours par le virus de la dengue sérotype 1 et précipité avec 7% de polyéthylèneglycol (cf exemple 1: purification de la protéine NS1 du virus de la dengue sérotype 1). La réactivité des sumageants des différents hybridomes vis à vis du surnageant de culture de cellules Vero non infectées sert de témoin de signal non spécifique.

### a₃- Réactivité des anticorps monoclonaux anti-NS1 par immunofluorescence indirecte sur des cellules Vero infectées par l'un des 4 sérotypes du virus de la dengue

Les cellules Vero sont infectées 40h avec l'un des 4 sérotypes du virus de la dengue:
sérotype 1 : souche FGA/89
sérotype 2: souche NG
sérotype 3: souche H87
sérotype 4: souche H241

Après 1 lavage avec une solution de PBS, les cellules sont fixées par une solution de paraformaldéhyde à 3% en PBS pendant 30 minutes à la température du laboratoire. Les cellules rincées en PBS sont ensuite perméabilisées par une solution de Triton X-100 à 0,5% en PBS pendant 10 minutes. Après rinçage en PBS, les cellules sont incubées pendant 1h avec les surnageants des différents hybridomes ayant réagi positivement en ELISA. Après 3 lavages avec du PBS, l'anticorps anti-IgG de souris marqué à la fluorescéine est ajouté et incubé pendant 1h. Après 3 lavages en PBS, les lames sont recouvertes d'une lamelle et observées sous microscope à fluorescence.

### a₄ Préparation des ascites monoclonales de souris

Les ascites monoclonales sont produites chez des souris Balb/C. Les souris reçoivent une injection intrapéritonéale de 0,5ml de pristane (2,6,10,14-tetramethyl-pentadecane Sigma) une semaine avant l'injection intrapéritonéale du clone d'hybridome sécrétant l'anticorps monoclonal. Les ascites sont prélevées au fur et à mesure de leur formation, centrifugées à 1500 rpm pendant 20 minutes et conservées à -20°C.

### a₅- Détermination de l'isotype des anticorps monoclonaux anti-NS1

La détermination de l'isotype de l'anticorps anti-NS1 s'effectue par ELISA à l'aide d'anticorps dirigés -contre les différentes sous-classes d'immunoglobulines murines: IgG1, IgG2a, IgG2b, IgG3. La détermination de la chaîne légère de l'immunoglobuline est réalisée suivant une méthodologie identique.

### a₆- Détermination de la constante d'affinité des anticorps monoclonaux anti-NS1 (B Friguet et al ., J.Immunol, (1985), 77, 305-319)

L'affinité d'un anticorps correspond à la concentration en antigène nécessaire pour saturer 50% des sites de l'anticorps. Une incubation est réalisée en milieu liquide entre l'anticorps à concentration constante et l'antigène à concentration décroissante pendant 1 nuit à 4°C afin d'atteindre l'équilibre de la réaction. La concentration en anticorps libre, après équilibre, est déterminée par un test ELISA: le mélange est déposé sur une plaque préalablement incubée avec l'antigène. Après une incubation de 20 minutes à 4°C (pour éviter un déplacement de l'équilibre), la révélation de l'ELISA se fait avec un anti-IgG dé souris couplé à la B galactosidase suivi de la réaction enzymatique. La constante de dissociation KD est ensuite déterminée.

### a₇ Réaction de compétition des différents anticorps monoclonaux anti-NS1

Cette réaction permet de déterminer la spécificité des anticorps monoclonaux vis à vis d'un même épitope ou d'épitopes différents. La détermination épitopique met en jeu la réactivité pour un antigène, d'un anticorps monoclonal non marqué et d'un deuxième anticorps monoclonal couplé à la biotine.

Le premier anticorps monoclonal non marqué est placé à concentration saturante (préalablement déterminée par ELISA) sur une plaque sur laquelle a été préalablement fixé l'antigène et incubé 2h à 37°C. Après 4 lavages en solution PT à 4°C, le deuxième anticorps monoclonal couplé à la biotine est ajouté et incubé 20 minutes à 4°C. Après 4 lavages en solution PT à 4°C, la solution de conjugué streptavidine marquée à la péroxydase est ajoutée et incubée 1h à 37°C. Après 4 lavages en solution PT, on révèle par une solution d'eau oxygénée en présence d'orthophénylènediamine. L'obtention d'un signal après lecture au spectrophotomètre indique que les épitopes reconnus par les 2 anticorps sont différents. Dans le cas inverse, les 2 anticorps monoclonaux sont dirigés contre le même épitope de l'antigène.

### b-purification des anticorps monoclonaux G18 et F22

Les anticorps G18 et F22 sont purifiés par immunoaffinité comme décrit dans l'exemple 3 .

### c- détection de la protéine NS1 circulante par un test ELISA-capture utilisant les anticorps monoclonaux

Les anticorps monoclonaux purifiés G18 et F22 sont mélangés dans une solution de PBS à une dilution donnée et mis à incuber une nuit à 4°C. Les étapes suivantes de ce test ELISA sont semblables à celles de l'exemple précédent.

### d- comparaison du test ELISA-capture utilisant l'approche monoclonale avec celui utilisant l'approche polyclonale

Un panel de sérum de Guyane française a été testé le même jour avec le test ELISA-capture utilisant l'approche monoclonale et celui utilisant l'approche polyclonale. Les sérums sont testés à différents dilutions: 10ème, 30ème et 90ème.

### 2. Résultats

### a- Caractéristiques des anticorps monoclonaux

Les résultats sont regroupés dans la figure 6.

Les anticorps G18 et F22 ont été sélectionné pour leur capacité à se fixer, avec une forte affinité, sur des épitopes différents de 1a protéine NSI. L'anticorps F22 est spécifique du sérotype 1, G 18 des sérotypes 1 et 3 du virus de la dengue.

### b- Utilisation des anticorps monoclonaux pour la capture d'antigène NS1.

Les résultats sont regroupés dans la figure 7.

Les anticorps monoclonaux sélectionnés non seulement reproduisent les résultats obtenus avec l'approche polyclonale mais ils présentent des réactivités plus marquées que les anticorps polyclonaux. L'outil monoclonal développé semble donc particulièrement adapté à l'utilisation diagnostique qui doit en être faite.

### Exemple 6: Mise en oeuvre de la technique ELISA-capture selon l'invention dans le cadre d'une infection par un autre sérotype du virus de la dengue ou un autre flavivirus

### 1. Matériel et méthodes

### a- Préparation des surnageants de culture

Les cellules Véro sont infectées soit par le virus dengue 2, soit par le virus de l'encéphalite japonaise ou le virus de la fièvre jaune. Les surnageants de culture sont ensuite préparés selon la méthode décrite dans l'exemple 1

### b-Purification des anticorps monoclonaux dirigés contre la protéine NS1 du virus de la fièvre jaune et de l'encéphalite japonaise

Les ascites monoclonales des anticorps 8G4, 1A5, et 2D10 (J.J. Schlesinger *et al., Virology,* (1983), **125**, 8-17) dirigés contre la protéine NS1 du virus de la fièvre jaune et des anticorps 171-2-2 et 70-14-20 dirigés contre la protéine NS1 du virus de l'encéphalite japonaise sont purifiées sur des billes de protéine A Sepharose CL-4B (Pharmacia Biotech). Ces ascites monoclonales sont mises à incuber une nuit à 4°C sur les billes de protéine A. Après 3 rinçages des billes en PBS/Tween 0,05%, les anticorps fixés sur les billes de protéine A sont élués avec une solution de tampon glycine pH=3. Ils sont ensuite concentrés par ultrafiltration et replacés dans un tampon PBS contenant de l'azide de sodium 1mM.

### c- Détection de la protéine NS1 dans les surnageants de culture du virus dengue 2

### c₁- Anticorps utilisés

L'étape de capture est réalisée avec un mélange des ascites des anticorps monoclonaux 3D1.4 et 1A12 (A.K.I. Falconar *et al., Arch. Virology,* (1994), **137**, 315-326). La reconnaissance de la protéine se fait ensuite avec un mélange de deux anticorps de lapin: le sérum obtenu après immunisation avec la protéine purifiée décrit dans l'exemple 3 et un sérum de lapin obtenu après immunisation avec les virus des quatre sérotypes de dengue.

### c₂- Procéde ELISA-capture

La technique utilisée est la même que celle décrite dans l'exemple 3

### d- Détection de la protéine NS1 dans les sumageants de culture du virus de l'encéphalite japonaise

### d₁- Anticorps utilisés

Les anticorps monoclonaux 171-2-2 et 70-14-20 purifiés sont utilisés pour l'étape de capture. La reconnaissance de la protéine se fait ensuite avec un mélange de deux sérums de lapins qui ont été immunisés préalablement avec des protéines recombinées de la protéine NS1 de l'encéphalite japonaise.

### d₂- Procéde ELISA-capture

La technique utilisée est la même que celle décrite dans l'exemple 3

### e-Détection de la protéine NS1 dans les surnageants de culture du virus de la fièvre jaune et les sérums de patients infectés par ce virus

### e₁- Anticorps utilisés

Les anticorps monoclonaux 8G4, 1A5, et 2D10 purifiés sont mélangés à une dilution donnée dans une solution de PBS et utilisés comme anticorps de capture. Le second anticorps spécifique de NS1 fièvre jaune utilisé provient d'un sérum de lapin immunisé préalablement contre la protéine NS1 du virus 17D de la fièvre jaune (J.J. Shlesinger *et al., J immunol. (1*985), **135**, 2805-2809).

### e₂- Procéde ELISA-capture

La technique utilisée est la même que celle décrite dans l'exemple 3

### 2. Résultats

La sécrétion de la protéine NS1 a précédemment été rapportée dans des cultures cellulaires *in vitro* infectées par différents flavivirus, le virus de la DEN2 (Winkler *et al., Virology* (1988), **162,** 187-196, Pryor *et al., Virology* (1993), **194,** 769-780), de l'encéphalite à tique (Lee *et al., J. Gen. Virol.* (1989), **70,** 335-343, Crooks *et al., J Chrom.* (1990), **502,** 59-68, Crooks *et al., J. Gen. Virol.* (1994), **75**. 3453-3460), de l'encéphalite japonaise (Mason, *Virology* (1989), **169,** 354-364, Fan *et al., Virology* (1990), **177**, 470-476), de l'encéphalite de la vallée de Murray (Hall *et al., J Virol. Meth.* (1991), **32,** 11-20) et de la fièvre jaune (Post *et al., Vir. Res. (1990),* **18,** 291-302). Comme ces résultats ont été obtenus par des techniques différentes de l'ELISA, nous avons recherché à mettre en évidence la protéine, par la technique ELISA-capture de la présente invention, dans des surnageants de cellules de mammifère infectées.

La protéine NS 1 est détectable dans les sumageants de culture des cellules Vero infectées soit par le virus DEN2, soit par le virus de l'encéphalite japonaise, soit par le virus de la fièvre jaune.

La protéine a aussi pu être mise en évidence par cette technique dans des sérums de patients infectés par le virus de la fièvre jaune comme le démontre les résultats rassemblés dans la figure 8. Parmis les 18 sérums généreusement fournis par Ch. Mathiot (Institut Pasteur de Dakar), 7 sont positifs en antigénémie NS1 et comme pour le virus DEN1, la détection de la protéine NS1 circulante semble indifférente à la présence d'IgM spécifiques de la fièvre jaune.

La technique ELISA-capture selon la présente invention permet de détecter de la protéine NS1 dans les surnageants de culture de cellules infectées par différents flavivirus et dans les sérums des patients infectés par le virus de la fièvre jaune. Elle pourrait avoir de ce fait une application diagnostique pour déceler une infection par d'autre flavivirus que le virus DEN1.

## Revendications

1. Méthode de détection précoce d'une infection flavivirale, **caractérisée en ce qu'**elle comprend la détection de la glycoprotéine non-structurale NS1 d'un flavivirus dans un échantillon biologique, pendant toute la durée de la phase clinique de l'infection, par une méthode immunologique mettant en oeuvre au moins deux anticorps identiques ou différents,
- le premier anticorps ou anticorps de capture de la glycoprotéine NS1 étant constitué par des anticorps choisis dans le groupe constitué par :
- des anticorps polyclonaux préalablement sélectionnés par immunocapture sur la protéine NS 1 dudit flavivirus, de forme hexamérique, et
- des mélanges d'anticorps monoclonaux anti-NS1 préalablement sélectionnés pour leur affinité élevée pour la protéine NS1 dudit flavivirus, de forme hexamérique, lesdits anticorps monoclonaux étant ensuite purifiés,
- le deuxième anticorps ou anticorps de révélation étant choisi dans le groupe constitué par :
- des anticorps polyclonaux dirigés contre la protéine NS 1 de forme hexamérique et
- un mélange d'anticorps monoclonaux dirigés contre une protéine NS 1 de forme hexamérique.

2. Méthode de détection selon la revendication 1, **caractérisée en ce que** l'infection flavivirale est une infection par le virus de la dengue.

3. Méthode de détection selon l'une quelconque des revendications 1 ou 2, **caractérisée en ce que** le premier anticorps est fixé sur un support solide convenable et le deuxième anticorps est éventuellement conjugué à un marqueur approprié.

4. Méthode de détection selon la revendication 3, **caractérisée en ce que** lorsque le deuxième anticorps n'est pas conjugué à un marqueur, sa liaison à la protéine NS1 fixée sur le support solide, est alors détectée par un troisième anticorps conjugué à un marqueur convenable.

5. Méthode de détection selon la revendication 4, **caractérisée en ce que** le troisième anticorps est conjugué à une enzyme.

6. Méthode de détection selon la revendication 5, **caractérisée en ce que** :
- le premier anticorps ou anticorps de capture est constitué par des anticorps polyclonaux de souris, sélectionnés par immunocapture sur la protéine NS 1 du virus de la dengue, ladite protéine étant sous forme hexamérique, et
- le deuxième anticorps ou anticorps de détection de la présence de NS1 dans l'échantillon biologique à analyser, est constitué par des anticorps polyclonaux de lapin immunisé par de la protéine NS1 du virus de la dengue de sérotype 1, ladite protéine étant sous forme hexamérique, la fixation dudit deuxième anticorps étant révélé par un troisième anticorps constitué par des anticorps, conjugués à la peroxydase et dirigés contre le deuxième anticorps.

7. Coffret de diagnostic précoce d'une infection flavivirale, **caractérisé en ce qu'**il comprend :
- au moins un anticorps de capture et au moins un anticorps de révélation tels que définis dans l'une quelconque des revendications 1 à 6,
- au moins un contrôle positif constitué par la protéine NS1 d'un flavivirus et/ou de différents sérotypes selon le flavivirus, ladite protéine, étant sous forme hexamérique, et
- au moins un contrôle négatif constitué par un sérum humain normal.

8. Coffret de diagnostic selon la revendication 7, **caractérisé en ce que** ladite protéine NS1 est obtenue à partir d'un surnageant de culture, soit de cellules de mammifères infectées, soit de cellules de mammifères transfectées par un plasmide recombiné, comportant le gène de la protéine NS1 d'un flavivirus ou un fragment dudit gène ou un fragment du génome flaviviral, lesdits fragments étant aptes à exprimer tout ou partie de la protéine NS1.

9. Coffret de diagnostic précoce d'une infection flavivirale selon l'une quelconque des revendications 7 ou 8, **caractérisé en ce que** la protéine NS1 est celle du virus de la dengue.

10. Coffret de diagnostic précoce d'une infection flavivirale selon l'une quelconque des revendications 8 ou 9, **caractérisé en ce que** ledit plasmide a été déposé auprès de la Collection Nationale de Cultures et de Microorganismes tenue par l'Institut Pasteur sous le numéro 1-2220, en date du 7 juin 1999.

11. Procédé de purification de la protéine NS1 d'un flavivirus, sous forme hexamérique, à partir d'un surnageant de culture, soit de cellules de mammifères infectées, soit de cellules de mammifères transfectées par un plasmide recombiné, comportant le gène de la protéine NS1 ou un fragment dudit gène ou un fragment du génome flaviviral, lesdits fragments étant aptes à exprimer la protéine NS1, **caractérisé en ce que**, préalablement à la purification de la protéine NS1 par les techniques classiques, il comprend une étape de séparation de la forme soluble de la protéine NS1 de la forme microparticulaire de ladite protéine, par traitement par un agent précipitant puis par centrifugation.

12. Composition immunogène, **caractérisée en ce qu'**elle comprend à titre de principe actif la protéine NS1 d'un flavivirus, de forme hexamérique, éventuellement associée à d'autres protéines, en association avec au moins un véhicule pharmaceutiquement acceptable.

13. Composition immunogène selon la revendication 12, **caractérisée en ce qu'**elle comprend au moins un mélange des protéines NS1 de forme hexamérique correspondant aux différents sérotypes du virus de la dengue.

14. Utilisation de la protéine NS1, de forme hexamérique ou d'un de ses systèmes d'expression, pour la préparation d'une composition immunogénique, apte à induire la production d'anticorps *in vivo.*

15. Utilisation d'au moins un anticorps monoclonal anti-NS1 présentant une affinité élevéee pour la protéine NS1 de forme hexamérique, ladite forme étant non dégradée, lesdits anticorps monoclonaux étant ensuite purifiés, et modifiés pour la fabrication d'un médicament apte à induire une immunisation passive.

16. Utilisation de la protéine NS1 de forme hexamérique, ladite forme étant non dégradée, pour sélectionner *in vitro* des anticorps spécifiques anti-NS1 aptes au diagnostic précoce d'une infection par un flavivirus.

17. Composition immunogène, **caractérisée en ce qu'**elle comprend un principe actif sélectionné dans le groupe constitué par :
- un polynucléotide capable d'exprimer tout ou partie de la protéine NS 1 du virus de la dengue quel que soit son sérotype et
- un système d'expression comprenant au moins un promoteur capable de faire exprimer chez l'hôte où elle est injectée, l'ADN codant pour la protéine NS1 du virus de la dengue quel que soit son sérotype, ledit gène exprimant ladite protéine,
en association avec au moins un véhicule pharmaceutiquement acceptable.

18. Procédé d'expression d'un polynucléotide codant pour la protéine NS1 d'un virus de la dengue, **caractérisé en ce qu'**il comprend l'expression d'un polynucléotide tel que défini à la séquence SEQ ID N° 1, associé à un promoteur dudit polynucléotide dans des cellules eucaryotes convenables.

19. Procédé de purification de la protéine NS1 selon la revendication 11, **caractérisé en ce que** le flavivirus est un virus de la dengue quel que soit son sérotype.

20. Procédé de purification de la protéine NS1 selon les revendications 11 et 19, **caractérisé en ce que** le flavivirus est un virus de la dengue de sérotype 1

## Patentansprüche

1. Verfahren zum frühen Nachweis einer Flavivirus-Infektion, **dadurch gekennzeichnet, dass** es den Nachweis des nicht-strukturellen Glycoproteins NS1 eines Flavivirus in einer biologischen Probe während der gesamten Dauer der klinischen Phase der Infektion durch ein immunologisches Verfahren unter Verwendung von mindestens zwei Antikörpern, die gleich oder unterschiedlich sind, umfasst, wobei
- der erste Antikörper oder Einfangantikörper des Glycoproteins NS1 durch Antikörper gebildet wird, die aus der Gruppe bestehend aus:
- polyklonalen Antikörpern, die vorher durch Immuneinfangen auf dem Protein NS1 des Flavivirus in Hexamerform selektioniert werden; und
- Gemischen monoklonaler Anti-NS1-Antikörper, die vorher bezüglich ihrer erhöhten Affinität für das Protein NS1 des Flavivirus in Hexamerform selektioniert werden, wobei die monoklonalen Antikörper dann gereinigt werden,
ausgewählt wird;
- der zweite Antikörper oder Entwicklungsantikörper aus der Gruppe bestehend aus
- polyklonalen Antikörpern, die gegen das Protein NS1 in Hexamerform gerichtet sind und
- einem Gemisch monoklonaler Antikörper, die gegen ein Protein NS1 in Hexamerform gerichtet sind, ausgewählt wird.

2. Nachweisverfahren nach Anspruch 1, **dadurch gekennzeichnet, dass** die Flavivirusinfektion eine Infektion durch das Virus des Dengue-Fiebers ist.

3. Nachweisverfahren nach Anspruch 1 oder 2, **dadurch gekennzeichnet, dass** der erste Antikörper auf einem zweckdienlichen festen Träger fixiert ist und der zweite Antikörper gegebenenfalls mit einem geeigneten Marker konjugiert ist.

4. Nachweisverfahren nach Anspruch 3, **dadurch gekennzeichnet, dass**, wenn der zweite Antikörper nicht an einen Marker konjugiert ist, seine Bindung an das Protein NS1, das auf dem festen Träger fixiert ist, durch einen dritten Antikörper, der mit einem zweckdienlichen Marker konjugiert ist, detektiert wird.

5. Verfahren nach Anspruch 4, **dadurch gekennzeichnet, dass** der dritte Antikörper mit einem Enzym konjugiert ist.

6. Nachweisverfahren nach Anspruch 5, **dadurch gekennzeichnet, dass**
- der erste Antikörper oder Einfangantikörper aus polyklonalen Mausantikörpern gebildet wird, die durch Immuneinfangen an dem Protein NS1 des Virus des Dengue-Fiebers selektioniert werden, wobei das Protein in Hexamerform vorliegt, und
- der zweite Antikörper oder Antikörper zum Nachweis des Vorliegen von NS1 in der zu analysierenden biologischen Probe aus polyklonalen Kaninchenantikörpern, die durch das Protein NS1 des Virus des Dengue-Fiebers, Serotyp 1, immunisiert sind, wobei das Protein in Hexamerform vorliegt, bestehen, wobei die Fixierung des zweiten Antikörpers durch einen dritten Antikörper entwickelt wird, der aus Antikörpern besteht, die an Peroxidase konjugiert sind und gegen den zweiten Antikörper gerichtet sind.

7. Kit zur frühen Diagnose einer Flavivirusinfektion, **dadurch gekennzeichnet, dass** er umfasst:
- mindestens einen Einfangantikörper und mindestens einen Entwicklungsantikörper, wie sie in einem der Ansprüche 1 bis 6 definiert sind,
- mindestens eine positive Kontrolle, die durch das Protein NS1 eines Flavivirus und/oder verschiedene Serotypen entsprechend dem Flavivirus gebildet wird, wobei das Protein in Hexamerform vorliegt, und
- mindestens eine negative Kontrolle, die aus einem normalen humanen Serum besteht.

8. Kit zur Diagnose nach Anspruch 7, **dadurch gekennzeichnet, dass** das Protein NS1 aus einem Kulturüberstand, entweder von infizierten Säugerzellen oder Säugerzellen, die durch ein rekombiniertes Plasmid transfiziert sind, welches das Gen des Proteins NS1 eines Flavivirus oder ein Fragment des Gens oder ein Fragment des Flavivirusgenoms trägt, wobei die Fragmente geeignet sind, das Protein NS1 ganz oder teilweise zu exprimieren, erhalten wird.

9. Kit zur frühen Diagnose einer Flavivirusinfektion nach Anspruch 7 oder 8, **dadurch gekennzeichnet, dass** das Protein NS1 das Virus des Dengue-Fiebers ist.

10. Kit zur frühen Diagnose einer Flavivirusinfektion nach Anspruch 8 oder 9, **dadurch gekennzeichnet, dass** das Plasmid bei der Collection Nationale de Cultures et de Microorganismes, unterhalten vom Institut Pasteur, mit der Nummer 1-2220 am 7. Juni 1999 hinterlegt wurde.

11. Verfahren zur Reinigung des Proteins NS1 eines Flavivirus in Hexamerform ausgehend vom einem Kulturüberstand entweder von infizierten Säugerzellen oder von Säugerzellen, die durch ein rekombiniertes Plasmid transfiziert sind, welches das Gen des Proteins NS1 oder ein Fragment dieses Gens oder ein Fragment des Flavivirusgenoms trägt, wobei die Fragmente fähig sind, das Protein NS1 zu exprimieren, **dadurch gekennzeichnet, dass** es vor der Reinigung des Proteins NS1 durch klassische Techniken eine Stufe der Abtrennung der löslichen Form des Proteins NS1 von der Mikroteilchenform des Proteins durch Behandlung mit einem Precipitationsmittel und danach eine Zentrifugation umfasst.

12. Immunogene Zusammensetzung, **dadurch gekennzeichnet, dass** sie als Wirkstoff das Protein NS1 eines Flavivirus in Hexamerform, gegebenenfalls assoziiert mit anderen Proteinen, in Kombination mit mindestens einem pharmazeutisch annehmbaren Vehikel umfasst.

13. Immunogene Zusammensetzung nach Anspruch 12, **dadurch gekennzeichnet, dass** sie mindestens ein Gemisch der Proteine NS1 in Hexamerform, die verschiedenen Serotypen des Virus des Dengue-Fiebers entsprechen, umfasst.

14. Verwendung des Proteins NS1 in Hexamerform oder eines seiner Expressionssysteme zur Herstellung einer immunogenen Zusammensetzung, die geeignet ist, die Antikörperproduktion in vivo zu induzieren.

15. Verwendung mindestens eines monoklonalen Anti-NS1-Antikörpers, der eine erhöhte Affinität für das Protein NS1 in Hexamerform aufweist, wobei die Form nicht abgebaut ist, die monoklonalen Antikörper dann gereinigt werden und zur Herstellung eines Medikaments, das geeignet ist, eine passive Immunisierung zu induzieren, modifiziert werden.

16. Verwendung des Proteins NS1 in Hexamerform, wobei die Form nicht abgebaut ist, um in vitro spezifische Anti-NS1-Antikörper zu selektionieren, welche für eine frühe Diagnose einer Infektion durch ein Flavivirus geeignet sind.

17. Immunogene Zusammensetzung, **dadurch gekennzeichnet, dass** sie einen Wirkstoff, ausgewählt aus der Gruppe bestehend aus:
- einem Polynucleotid, das fähig ist, das Protein NS1 des Virus des Dengue-Fiebers, ganz gleich welcher sein Serotyp ist, zu exprimieren, und
- einem Expressionssystem, das mindestens einen Promotor, der fähig ist, bei einem Wirt, in den sie injiziert ist, die DNA, die für das Protein NS1 des Virus des Dengue-Fiebers, ganz gleich welcher sein Serotyp ist, codiert, das Gen, dass das Protein exprimiert, umfasst,
in Kombination mit mindestens einem pharmazeutisch annehmbaren Vehikel umfasst.

18. Verfahren zur Expression eines Polynucleotids, das für das Protein NS1 eines Virus des Dengue-Fiebers codiert, **dadurch gekennzeichnet, dass** es die Expression eines Polynucleotids, wie es in der Sequenz SEQ ID NO:1 definiert ist, verbunden mit einem Promotor des Polynucleotids, in zweckdienlichen Eukaryontenzellen umfasst.

19. Verfahren zur Reinigung des Proteins NS1 nach Anspruch 11, **dadurch gekennzeichnet, dass** das Flavivirus ein Virus des Dengue-Fiebers, ganz gleich welcher sein Serotyp ist, ist.

20. Verfahren zur Reinigung des Proteins NS1 nach den Ansprüchen 11 und 19, **dadurch gekennzeichnet, dass** das Virus ein Virus des Dengue-Fiebers, Serotyp I, ist.

## Claims

1. A method for the early detection of a flaviviral infection, **characterized in that** it comprises detecting the NS1 nonstructural glycoprotein of a flavivirus in a biological sample, throughout the duration of the clinical phase of the infection, by an immunological method using at least two antibodies, which may be identical or different,
- the first antibody or antibody for capturing the NS1 glycoprotein consisting of antibodies chosen from the group consisting of:
- polyclonal antibodies preselected by immunocapture on the NS1 protein of said flavivirus, in the hexameric form, and
- mixtures of anti-NS1 monoclonal antibodies preselected for their high affinity for the NS1 protein of said flavivirus, in the hexameric form, said monoclonal antibodies then being purified,
- the second antibody or revelation antibody being chosen from the group consisting of:
- polyclonal antibodies directed against the NS1 protein in the hexameric form, and
- a mixture of monoclonal antibodies directed against a NS1 protein in the hexameric form.

2. The detection method as claimed in claim 1, **characterized in that** the flaviviral infection is an infection with the dengue virus.

3. The detection method as claimed in either of claims 1 and 2, **characterized in that** the first antibody is attached to a suitable solid support and the second antibody is optionally conjugated to a suitable label.

4. The detection method as claimed in claim 3, **characterized in that** when the second antibody is not conjugated to a label, its binding to the NS1 protein attached to the solid support is then detected with a third antibody, conjugated to a suitable label.

5. The detection method as claimed in claim 4, **characterized in that** the third antibody is conjugated to an enzyme.

6. The detection method as claimed in claim 5, **characterized in that**:
- the first antibody, or capture antibody, consists of mouse polyclonal antibodies selected by immunocapture on the NS1 protein of the dengue virus, said protein being in the hexameric form, and
- the second antibody, or antibody for detecting the presence of NS1 in the biological sample to be analyzed, consists of polyclonal antibodies from a rabbit immunized with the NS1 protein of dengue virus serotype 1, said protein being in the hexameric form, the attachment of said second antibody being revealed with a third antibody, consisting of antibodies conjugated to peroxidase and directed against the second antibody.

7. A boxed set for the early diagnosis of a flaviviral infection, **characterized in that** it comprises:
- at least one capture antibody and at least one revelation antibody as defined in any one of claims 1 to 6,
- at least one positive control consisting of the NS1 protein of a flavivirus and/or of various serotypes depending on the flavivirus, said protein being in the hexameric form, and
- at least one negative control consisting of a normal human serum.

8. The boxed set for diagnosis as claimed in claim 7, **characterized in that** said NS1 protein is obtained from a culture supernatant either from infected mammalian cells or from mammalian cells transfected with a recombined plasmid comprising the gene of the NS1 protein of a flavivirus or a fragment of said gene or a fragment of the flaviviral genome, said fragments being capable of expressing all or part of the NS1 protein.

9. The boxed set for the early diagnosis of a flaviviral infection as claimed in either of claims 7 and 8, **characterized in that** the NS1 protein is that of the dengue virus.

10. The boxed set for the early diagnosis of a flaviviral infection as claimed in either of claims 8 and 9, **characterized in that** said plasmid was deposited with the Collection Nationale de Cultures et de Microorganismes [National collection of cultures and microorganisms] held by the Institut Pasteur under the number 1-2220, dated June 7, 1999.

11. A method for purifying the NS1 protein of a flavivirus, in the hexameric form, from a culture supernatant either of infected mammalian cells or of mammalian cells transfected with a recombined plasmid comprising the gene of the NS1 protein or a fragment of said gene or a fragment of the flaviviral genome, said fragments being capable of expressing the NS1 protein, **characterized in that**, prior to the purification of the NS1 protein using conventional techniques, it comprises a step for separating the soluble form of the NS1 protein from the microparticulate form of said protein, by treatment with a precipitating agent and then by centrifugation.

12. An immunogenic composition, **characterized in that** it comprises, as the active principle, the NS1 protein of a flavivirus, in the hexameric form, optionally associated with other proteins, in combination with at least one pharmaceutically acceptable vehicle.

13. The immunogenic composition as claimed in claim 12, **characterized in that** it comprises at least one mixture of the NS1 proteins in the hexameric form corresponding to the various dengue virus serotypes.

14. The use of the NS1 protein in the hexameric form, or of a system for the expression thereof, for preparing an immunogenic composition capable of inducing the production of antibodies *in vivo.*

15. The use of at least one monoclonal anti-NS1 antibody having a high affinity for the NS1 protein in the hexameric form, said form being nondegraded, said monoclonal antibodies then being purified and modified, for manufacturing a medicinal product capable of inducing passive immunization.

16. The use of the NS1 protein in the hexameric form, said form being nondegraded, for selecting *in vitro* specific anti-NS1 antibodies able to diagnose an infection with a flavivirus, at an early stage.

17. An immunogenic composition, **characterized in that** it comprises an active principle selected from the group consisting of:
- a polynucleotide capable of expressing all or part of the NS1 protein of the dengue virus, whatever its serotype,
- an expression system comprising at least one promoter capable of expressing, in the host into which it is injected, a DNA encoding the NS1 protein of the dengue virus, whatever its serotype, said gene expressing said protein,
in combination with at least one pharmaceutically acceptable vehicle.

18. A method for expressing a polynucleotide encoding the NS1 protein of a dengue virus, **characterized in that** it comprises the expression of a polynucleotide as defined in the sequence SEQ ID No. 1, associated with a promoter for said polynucleotide, in suitable eukaryotic cells.

19. The method for purifying the NS1 protein as claimed in claim 11, **characterized in that** the flavivirus is a dengue virus, whatever its serotype.

20. The method for purifying the NS1 protein as claimed in claims 11 and 19, **characterized in that** the flavivirus is a dengue virus serotype 1.
